# EUROPEAN PATENT SPECIFICATION

(11) **EP 1 478 373 B1**
(45) Date of publication and mention of the grant of the patent: **04.10.2006**
(21) Application number: 02780174.5
(22) Date of filing: 15.11.2002
(51) Int. Cl.: A61K 31/714, A61K 31/70

(54) **PHARMACEUTICAL COMPOSITIONS COMPRISING ONE OR MORE STEROIDS ONE OR MORE TETRAHYDROFOLATE COMPONENTS AND VITAMIN B12**
PHARMAZEUTISCHE ZUSAMMENSETZUNG, ENTHALTEND EIN ODER MEHRERE STEROIDE, EINE ODER MEHRERE TETRAHYDROFOLATVERBINDUNGEN UND VITAMIN B12
COMPOSITIONS PHARMACEUTIQUES CONTENANT UN OU PLUSIEURS STEROIDES, UN OU PLUSIEURS TETRAHYDROFOLATES ET DE LA VITAMINE B12

(30) Priority: 21.02.2002 EP 02075695
(43) Date of publication of application: 24.11.2004
(73) Proprietor: Schering Aktiengesellschaft, 13353 Berlin (DE)
(72) Inventor: COELINGH BENNINK, Herman, Jan, Tijmen, NL-3971 BE Driebergen (NL)
(74) Representative: van Westenbrugge, Andries
(86) International application number: PCT/NL2002/000741
(87) International publication number: WO 2003/070255

(56) References cited:
- WO-A-01/24772
- WO-A-02/34199
- WO-A-98/04248
- WO-A-99/53910
- WO-A-99/65337
- KORNBERG A ET AL: "FOLIC ACID DEFICIENCY MEGALOBLASTIC ANEMIA AND PERIPHERAL POLYNEUROPATHY DUE TO ORAL CONTRACEPTIVES" ISRAEL JOURNAL OF MEDICAL SCIENCES, vol. 25, no. 3, 1989, pages 142-145, XP002197742 ISSN: 0021-2180
- WEBB J L: "NUTRITIONAL EFFECTS OF ORAL CONTRACEPTIVE USE A REVIEW" JOURNAL OF REPRODUCTIVE MEDICINE, vol. 25, no. 4, 1980, pages 150-156, XP002197743 ISSN: 0024-7758
- RAMSAY I D ET AL: "REDUCED SERUM VITAMIN B-12 LEVELS DURING ORAL CYPROTERONE ACETATE AND ETHYNYLESTRADIOL THERAPY IN WOMEN WITH DIFFUSE ANDROGEN-DEPENDENT ALOPECIA" CLINICAL AND EXPERIMENTAL DERMATOLOGY, vol. 15, no. 4, 1990, pages 277-281, XP002197794 ISSN: 0307-6938
- RANG: "Pharmacology" 1999 , CHURCHILL LIVINGSTON , EDINBURGH, LONDON XP002230531 "Folic acid" and "Vitamin B12" page 332 -page 333

## Description

### TECHNICAL FIELD OF THE INVENTION

The present invention relates to a pharmaceutical kit comprising a plurality of oral dosage units for use in a hormonal contraceptive method or hormone replacement therapy in mammalian females, said kit comprising at least 10 dosage units containing: one or more steroids selected from the group consisting of estrogens and progestogens; one or more tetrahydrofolate components; and vitamin B12.

Other aspects of the invention concern a hormonal contraceptive method and a method of hormone replacement therapy in mammalian females, said methods comprising the at least once daily oral administration of one or more of dosage units to a mammalian female to provide steroids in an effective amount to inhibit ovulation and/or to prevent or suppress symptoms of hypogonadism, and wherein the dosage units additionally contain one or more tetrahydrofolate components and vitamin B12.

### BACKGROUND OF THE INVENTION

The repeated oral administration of hormonal preparations, in particular in the context of hormonal contraception or hormone replacement therapy, has been associated with a depletion of folate (Martindale, The Complete Drug Reference, MICROMEDEX® Healthcare Series Vol. 111, expiration date 3/2002). Some reports have also made mention of a decrease in vitamin B 12 in users of oral contraceptives (e.g. Martindale).

Kornberg et al. (Israel Journal of Medical Sciences (1989), no.3, 142-145 describe a case study in which a 34-year old woman developed megaloblastic anemia and peripheral polyneuropathy following the use of oral contraceptives for 4 years. The author conclude that both the complete recovery after therapy with the vitamins, and the absence of vitamin B12 and folate deficiency, suggest that the vitamin deficiencies were caused by the oral contraceptive and resulted in the rare combination of megaloblastic anemia and polyneuropathy.

In Pharmacology (1999), pages 332-333 it is observed that for activity, folate must be in the tetrahydro form in which it is maintained by the enzyme dihydrofolate reductase. Furthermore, it is stated that folinic acid, a synthetic tetrahydrofolic acid, is converted much more to the polyglutamine form that folic acid. Unwanted effects are reported to possibly occur in the presence of B12 deficiency, because if vitamin B12 deficiency is treated with folic acid, the blood picture may improve and give the appearance of cure while the neurological levels get worse.

WO 99/53910 describes folate containing pharmaceutical compositions comprising either an oral contraceptive or a hormone replacement composition. These compositions are intended for use in methods for delivering folate to subjects afflicted with, or at an increased risk of becoming afflicted with, a folate treatable disorder. WO 99/53910 states that in pregnant women correction of low folate levels takes at least 2 months, and that reserves can last as little as a few weeks. It is also noted therein that supplementation of folate immediately before discontinuing oral contraceptive use or immediately after positive pregnancy test results may be insufficient to optimally protect the developing fetus. Furthermore it is stated that decreases of folate levels among oral contraceptive users pose an additional risk for such users who become pregnant within three to six months following discontinuation of use.

The combined administration of folate and an oral contraceptive or a hormone replacement composition as described in WO 99/53910 offers the advantage that it helps to prevent folate deficiency in users of oral contraceptives and hormone replacement compositions.

However, the incorporation of folate in oral contraceptives and hormone replacement compositions poses a serious health risk in that it will suppress symptoms of vitamin B12 deficiency such as megaloblastic anaemia. The haematologic abnormalities seen with a vitamin B 12 deficiency will respond to treatment with folate alone. However, the neuropsychiatric abnormalities caused by the vitamin B12 deficiency will not be corrected and may indeed by worsened. For example, the administration of folate to a subject, suffering from megaloblastic anaemia as a result of vitamin B 12 deficiency, will mask the early symptoms, allowing neurological symptoms like ataxia and paresthesia (Combined System Disease) to occur at a later stage.

Vitamin B12 deficiency is a multi-system disorder with an extremely varied clinical presentation which has been thought to occur in 0.4% of the US-population. Symptoms of vitamin B12 deficiency include significant anaemia, displayed, for example, in decreased haematocrit or haemoglobin, with macrocytic red blood cells, or neurologic symptoms of peripheral neuropathy and/or ataxia. The haematological abnormalities seen are due to intracellular folate deficiency since folate is required for a number of essential enzymatic reactions involved in DNA and RNA synthesis and since the form of folate in serum (5-formyltetrahydrofolate) must be metabolised to tetrahydrofolate by the vitamin B12-dependent enzyme methionine synthase before it can be utilised by the RNA- and DNA-related enzymes.

The incidence of folate deficiency in the population is unknown, but has been thought to occur commonly in individuals with various degrees of alcoholism, in individuals suffering from malabsorption or malnutrition, in females using hormonal contraceptives, in pregnant women and in some cancer patients. The common way to treat or prevent folate deficiency is to orally administer folate. In order to alleviate or prevent symptoms of folate deficiency, folates have to be metabolised to their metabolically active form in a number of steps. Following absorption, folate is reduced to dihydrofolate and then to tetrahydrofolate (THF) via folate and dihydrofolate reductase. Both of these enzymes require NADPH (niacin dependent) as a cofactor. Subsequently, serine combines with pyridoxal-5'-phosphate to transfer a hydroxymethyl group to THF. This results in the formation of 5, 10-methylenetetrahydrofolate and glycine. This molecule is of central importance, being the precursor of the metabolically-active 5-methyltetrahydrofolate, which is involved in homocysteine metabolism, and methylidynetetrahydrofolate (involved in purine synthesis), as well as functioning on its own in the generation of thymine side chains for incorporation into DNA. The oral bioavailability of folic acid has been shown to be widely variable. The literature contains reports of individuals having poor intestinal uptake of folic acid who respond normally to intramuscular injection of folic acid. The metabolisation of orally administered folate to its active metabolites is known to be affected by various physiological, nutritional and pharmaceutical factors. In particular, it is known that the reduction of folates to THF is hampered by external factors, such as the use of hormonal contraceptives and certain drugs (e.g. methotrexate, 5-florouracil, sulfasalazine, diphenylhydantoin, trimethoprim, pyrimethamine and sulphonamides). Thus, supplementation of folic acid or folate to female users of hormonal contraceptives suffers from the drawbacks that (a) it is an inefficient way of restoring normal serum folate levels and (b) more importantly, that the efficacy of such supplementation, due to individual differences in folate metabolisation, varies from individual to individual.

### SUMMARY OF THE INVENTION

The primary objective of the present invention is to realise the benefits of folate supplementation in methods of hormonal contraception and hormone replacement therapy without the aforementioned negative consequences for subj ects suffering from vitamin B 12 deficiency. The inventors have found that this objective can be achieved very effectively and reliably through the combined co-administration of tetrahydrofolate and vitamin B 12 in the context of an oral contraceptive method or a method of hormone replacement therapy.

In addition, in the present method, prevention of folate deficiency is achieved in a very effective and reliable manner through the administration of a tetrahydrofolate selected from the group consisting of (6S)-tetrahydrofolic acid, 5-methyl-(6S)-tetrahydrofolic acid, 5-formyl-(6S)-tetrahydrofolic acid, 10-formyl-(6R)-tetrahydrofolic acid, 5,10-methylene-(6R)-tetrahydrofolic acid, 5,10-methenyl-(6R)-tetrahydrofolic acid, 5-formimino-(6S)-tetrahydrofolic acid, pharmaceutically acceptable salts of these tetrahydrofolic acids and glutamyl derivatives of these tetrahydrofolic acids. Unlike folates, the physiological effect of the aforementioned tetrahydrofolates is predictable and reliable as it is not influenced by external factors such as, in particular, the concurrent administration of an oral contraceptive.

### DETAILED DESCRIPTION OF THE INVENTION

Accordingly, one aspect of the invention relates to a kit for use in a hormonal contraceptive method or hormone replacement therapy in mammalian females, said kit comprising at least 10 oral dosage units containing at least 1 µg of one or more steroids selected from the group consisting of estrogens and progestogens; at least 0.1 mg of one or more tetrahydrofolate components selected from the group consisting of of (6S)-tetrahydrofolic acid, 5-methyl-(6S)-tetrahydrofolic acid, 5-formyl-(6S)-tetrahydrofolic acid, 10-formyl-(6R)-tetrahydrofolic acid, 5,10-methylene-(6R)-tetrahydrofolic acid, 5,10-methenyl-(6R)-tetrahydrofolic acid, 5-formimino-(6S)-tetrahydrofolic acid, pharmaceutically acceptable salts of these tetrahydrofolic acids and glutamyl derivatives of these tetrahydrofolic acids; and at least 0.1 mg vitamin B 12.

Throughout this document the term "folate" encompassed folic acid as well as salts of folic acid. Similarly, the term "tetrahydrofolate" refers to tetrahydrofolic acids as well as salts of these acids. In a particularly preferred embodiment of the invention, the one or more tetrahydrofolate components are selected from the group consisting of 5-formyl-tetrahydrofolic acid (folinic acid), 5-methyl-tetrahydrofolic acid, as well as pharmaceutically acceptable salts and glutamyl derivatives of these acids. Even more preferably the one or more tetrahydrofolate components are selected from the group consisting of folinic acid and pharmaceutically acceptable salts and glutamyl derivatives of folinic acid. Most preferably the tetrahydrofolate component is folinic acid.

Folinic acid (5-formyl-tetrahydrofolic acid or leucovorin acid) has long been used in therapeutic doses for several diseases. Examples include rescue from the toxicity of methotrexate chemotherapy, and the synergistic combination with fluorouracil for treatment of various cancers. It is also given to treat acute anemia. 5-Methyl-tetrahydrofolic acid in high doses (for example, 50 mg/day) has been patented for treatment of depression and other neurological disorders (EP382019 and EP388827 to Le Grazie 1990, and EP482493 to Le Greca 1992).

The term vitamin B12 is used to describe compounds of the cobalt corrinoid family, in particular those of the cobalamin group. The most used compound of this group is cyanocobalamin and as such the term vitamin B12 is sometimes used to refer to cyanocobalamin. In this specification the term vitamin B12 should be attributed its broad meaning so as to include all cobalt corrinoids of the cobalamin group, which include in particular, cyanocobalamin, hydroxocobalamin, methylcobalamin and nitrocobalamin. The present invention encompasses the use of vitamin B 12 *per se* as well as precursors of vitamin B12 that are capable of releasing vitamin B12 *in vivo* when used in accordance with the present method and metabolites of vitamin B12 (e.g. a conjugate with a polypeptide) that display the same *in vivo* functionality as vitamin B12, in particular in terms of the ability to alleviate symptoms of vitamin B12 deficiency.

Vitamin B12 deficiency may occur in otherwise healthy individuals with intestinal absorption problems (malabsorption) and several other conditions. It may also result from the use of certain medications. Furthermore vitamin B12 deficiency is not unusual in vegans and vegetarians.

Examples of estrogens that may suitably be used in the present dosage units include ethinyl estradiol, mestranol, quinestranol, estradiol, estrone, estran, estriol, estetrol, conjugated equine estrogens and precursors thereof that are capable of releasing such an estrogen *in vivo* when used in the present method.

Progestogens that may suitably be incorporated in the present dosage units include levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagestone, chlormadinone, cyproterone, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone, megestrol, melengestrol, nomegestrol, norethindrone (=norethisterone), norethynodrel, norgestrel (includes d-norgestrel and dl-norgestrel), norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime and precursors of these compounds. Preferably the progestogen used in the progestogenic phase is selected from the group consisting of levonorgestrel, norgestimate, norethisterone, drospirenone, dydrogesterone as well as precursors thereof that are capable of releasing such a progestogen *in vivo* when used in the present method.

The present kit preferably comprises at least 10 oral dosage units containing from 2 µg to 30 mg of the one or more steroids; from 0.2 to 15 mg of the one or more tetrahydrofolate components and from 0.2 to 20 mg vitamin B12. Examples of suitably oral dosage units include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

The present kit can suitably take the form of container or a strip comprising the plurality of oral dosage units. In case of a strip, the daily (or other periodic) dosages can be arranged for proper sequential administration. In a preferred embodiment, the invention provides a kit comprising a pharmaceutical package containing a plurality of dosage units, adapted for successive daily administration.

The present kit may suitably be used in a variety of oral contraceptive methods. A well-known oral contraceptive regimen uses so called monophasic preparations that contain a constant amount of an estrogen and a progestogen throughout the administration cycle. Newer preparations known as bi- or triphasic preparations have varying levels of estrogen and progestogen; in most cases consisting of relatively constant levels of estrogen with a stepwise increase in progestogen throughout the cycle. Mono-, bi- and triphasic contraceptives are commonly referred to as combined contraceptives.

Virtually all combined contraceptives have in common that they are based on a regimen which involves an administration-free interval of about 7 days whereby withdrawal bleeding, simulating the natural menses, occurs. Thus 21 day intervals of hormone administration alternate with 7 days during which no hormones are administered.

As an alternative to the aforementioned contraceptive methods, the so called sequential method has been proposed. Typical of the sequential contraceptive method is that it comprises two consecutive phases, i.e. one phase during which estrogen and no progestogen is administered and another phase during which a combination of estrogen and progestogen is administered. The first sequential methods, like the aforementioned combined contraceptives, made use of an administration free interval of about 7 days. More recently, sequential methods have been proposed which do not include such an administration-free (or placebo) period, meaning that estrogen is administered throughout the full cycle and that progestogen is co-administered during only part of that cycle. WO 95/17895 (Ehrlich et al.) describes such an uninterrupted sequential method.

Another example of an oral contraceptive method that employs uninterrupted continuous administration is the so called continuous combined method, which employs the combined administration of estrogen and progestogen during a period of more than 28 days, in particular more than 2 months. Yet another example of an oral contraceptive method that employs uninterrupted continuous administration is the progestogen only method, which employs continuous administration of a progestogen without an estrogen during a period or more than 28 days, especially more than 2 months.

In case the present kit is used in a contraceptive method that employs an interval during which no steroids are administered, it is preferred to continue the administration of the tetrahydrofolate component and vitamin B12 (and other optional vitamins, such as vitamin B6) during said interval. Consequently, said kit preferably comprises one or more dosage units, preferably from 3-8 dosage units, that contain one or more tetrahydrofolate components and vitamin B 12 in the amounts indicated herein, but that contain virtually no progestogen or estrogen.

The continuous, uninterrupted administration of the one or more tetrahydrofolate components and vitamin B 12 is found to be more effective in preventing and treating deficiencies of either or both components than a protocol in which said administration is interrupted for several days during each (4 weekly) cycle. Consequently, in a preferred embodiment the method comprises the essentially continuous administration of the one or more tetrahydrofolate components and vitamin B12 (and other optional vitamins such as vitamin B6) during a time interval of at least 40 days, preferably at least 90 days. In another advantageous embodiment the method comprises an interval of 3-8 days during which the one or more tetrahydrofolate components and vitamin B12 are administered, but during which no estrogen or progestogen is administered. As mentioned above, it is advantageous to continue the uninterrupted administration of the tetrahydrofolate component and vitamin B12 even if a contraceptive protocol (e.g. in case of a combined contraceptive) requires that during a particular interval no estrogen or progestogen are to be administered.

Contraceptive methods that do not employ administration free intervals (or placebo's) are more likely to cause folate depletion than methods that do make use of such intervals. Hence, the advantages of the present invention are particularly pronounced in oral contraceptives that do not employ regular, e.g. 4-weekly, administration free intervals. Similarly, the present invention offers significant benefits for methods of hormone replacement therapy, which make use of continuous uninterrupted administration of steroids, particularly of an estrogen in combination with a progestogen. Accordingly, in a preferred embodiment all of the dosage units within the present kit comprise the one or more steroids, the one or more tetrahydrofolate components and vitamin B12 in the indicated amounts, meaning that the kit does not comprise any placebo's.

Another aspect of the present invention relates to a hormonal contraceptive method comprising the at least once daily oral administration of one or more steroid containing dosage units to a mammalian female so as to provide steroids in an effective amount to inhibit ovulation, and wherein the dosage units additionally contain at least 0.1 mg of the one or more tetrahydrofolate components and at least 0.1 mg vitamin B12.

Yet another aspect of the invention relates to a method of hormone replacement therapy in peri-menopausal, menopausal or post menopausal mammalian females, said method comprising the at least once daily oral administration of one or more steroids containing dosage units to the female so as to provide steroids in an effective amount to prevent or suppress symptoms of hypogonadism, and wherein the dosage units additionally contain at least 0.1 mg of the one or more tetrahydrofolate components and at least 0.1 mg vitamin B12.

The steroids used in accordance with the above methods are preferably selected from the group consisting of estrogens and progestogens. Examples of suitable estrogens and progestogens have been presented above. Preferably the estrogen is selected from the group consisting of ethinyl estradiol, 17β-estradiol, estetrol and precursors thereof. The progestogens are preferably selected from the group consisting of levonorgestrel, norgestimate, norethisterone, drospirenone, dydrogesterone, trimegestone and precursors thereof.

The benefits of the present invention are particularly pronounced in case the tetrahydrofolate component and vitamin B12 are co-administered together with ethinyl estradiol because ethinyl estradiol has a particularly depressing effect on serum folate concentration. Ethinyl estradiol is the estrogen used in virtually all oral contraceptives that are on the market todate. In contrast, ethinyl estradiol is hardly used in hormone replacement therapy. According to a particularly preferred embodiment of the invention the dosage units contain between 3 and 40 µg, preferably between 10 and 30 µg ethinyl estradiol.

The main objective of the inclusion of both a tetrahydrofolate component and vitamin B12 is to prevent or remedy deficiency of either of these vitamins. Folate deficiency may typically result from chronic administration of the aforementioned steroids. Thus, the dosage units are advantageously administered to provide a therapeutically effective amount of the one or more tetrahydrofolate components to prevent or remedy folate deficiency. The present method is particularly effective in preventing or suppressing folate deficiency resulting from prolonged administration of the aforementioned steroids. Preferably the vitamin B12 is also administered to provide a therapeutically effective amount of vitamin B12 to prevent or remedy deficiencies of vitamin B12.

Vitamin deficiencies are generally determined by measurement of serum levels. Normal serum vitamin B12 levels are 211-911 pg/ml, with levels of less than about 100 pg/ml being said to indicate clinically significant deficiency. However, serum vitamin B 12 levels are a relatively insensitive determinant of vitamin B 12 deficiency in that only 50% of patients with clinically confirmed vitamin B12 deficiency have levels less than 100 pg/ml, 40% are 100-200 pg/ml, and at least 5-10% have values in the 200-300 pg/ml range. Diagnosis is further complicated by the fact that 2.5% of normal subjects have low serum vitamin B12 levels, with no evidence of vitamin B12 deficiency.

Normal serum folate levels are above 2.8 ng/ml, with levels less than 2.8 ng/ml indicating the possibility of clinically significant deficiency. Like vitamin B12 serum levels, however, serum folate levels are a relatively insensitive measure in that only 50-75% of patients with folate deficiency have levels less than 2.8 ng/ml. The development of sensitive serum metabolite assays for homocystein (HC), cystathionine (CT), methylmalonic acid (MMA), and 2-methylcitric acid (2-MCA) has allowed the relationship between metabolite levels and vitamin deficiencies to be investigated (Stabler et al. (1987) Anal. Biochem. 162: 185-196; Stabler et al. (1986) J. Clin. Invest. 77: 1606-1612; Stabler et al. (1988) J. Clin. Invest. 81: 466-474).

It has been found that elevated serum levels of HC and MMA are clinically useful tests of functional intracellular deficiencies of vitamin B12 and folate, with elevated HC levels seen with both vitamin B12 and folate deficiencies, and elevated MMA levels seen with a vitamin B12 deficiency (Allen et al. (1990) Am. J. Hematol. 34: 90-98; Lindenbaum et al. (1990) Am. J. Hematol. 34: 99-107; Lindenbaum et al. (1988) N. Engl. J. Med. 318: 1720-1728; Beck (1991) in Neuropsychiatric Consequences of Cobalamin Deficiency, Mosby Year Book 36: 33-56; Moelby et al. J Intern Med (1990) 228: 373-378; Ueland and Refsum (1989) J. Lab. Clin. Med. 114: 473-501; Pennypacker et al. J Am Geriatr Soc (1992) 40: 1197-1204). Increased serum levels of CT are seen in both deficiencies and 2-MCA is elevated in vitamin B12 deficiency.

In a preferred embodiment of the present methods, the one ore more tetrahydrofolate components and vitamin B12 are administered to a female with elevated serum levels of homocysteine, cysthathionine, methylmalonic acid and/or 2-methylcitric acid, in a therapeutically effective amount to significantly reduce the serum level of at least one of those substances. In a particularly preferred embodiment the present method restores the levels of all these substances to normal serum levels.

The present methods preferably comprise an administration regimen which provides to the female at least 18 consecutive daily dosages of:
from 2 µg to 30 mg of the one or more steroids;
from 0.2 to 15 mg of the one or more tetrahydrofolate components and
from 0.2 to 20 mg vitamin B12.

In another preferred embodiment, the methods according to the invention comprise an administration regimen that provides to the female at least 18 consecutive daily dosages of estrogen in an amount equivalent to 3-40 µg ethinyl estradiol and/or progestogen in an amount equivalent to 30 - 750 µg levonorgestrel.

As mentioned herein before, the advantages of the invention are particularly pronounced in hormonal replacement methods and methods of oral contraception that employ continuous uninterrupted administration of one or more steroids. Hence, the method according to the invention preferably comprises essentially continuous administration of the steroid containing dosage units during a time interval of at least 40 days, preferably at least 90 days.

The term "continuous" when used in relation to the administration of one or more active principles, means that said one or more active principles are administered at relatively regular intervals, with no (therapeutically) significant interruptions. Naturally, minor interruptions may occur that do not affect the overall effectiveness of the present method, and indeed such aberrations are encompassed by the present invention. In a preferred embodiment, and more arithmetically, an administration regimen is deemed to be continuous if the longest interval between 2 subsequent administrations is not more than 3.5 times as long as the average interval. Even more preferably said longest interval is not more than 2.5 times as long as the average interval.

Similarly to a vitamin B12 deficiency, vitamin B6 (pyridoxine) deficiencies also result in haematologic as well as neuropsychiatric abnormalities. Vitamin B6 is required for the first step in haem synthesis and serves a major role in transamination reactions of amino acid metabolism, in decarboxylations, and in the synthesis of the neuroactive amines histamine, tyramine, serotonin, and γ-aminobutyric acid. Clinical manifestations include microcytic hypochromic anaemia, characteristic skin changes of dermatitis and acrodynia, muscular weakness, and a variety of neuropsychiatric abnormalities including hyperirritability, epileptiform convulsions, depression and confusion (Newberne and Conner (1989) in Clinical Biochemistry of Domestic Animals, Academic Press, San Diego, pp. 796-834).

The human body typically contains between 40 and 150 mg vitamin B6. The required daily intake is 1-2 mg. During pregnancy it is usually advised to consume higher amounts of vitamin B6. A normal diet would normally satisfy this increased requirement, but a diet analysis of 26 pregnant females in the United States showed that only one female consumed more than 2.5 mg of vitamin B6 per day. Studies conducted in the US and Sweden (Hamfelt and Tuveno Clin Chem Acta (1972) 41: 287 and Lumeng et al. Am J Clin Nutr (1976) 29: 1376-1383) suggest that for pregnant females a daily intake of around 10 mg vitamin B6 would be advisable. It is believed that the increased requirement of vitamin B6 may be explained by the important role of this vitamin in fetal development. This important role is illustrated by the finding that in pregnant human females the vitamin B6 concentration in blood taken from the umbilical cord is higher than in the maternal blood. Furthermore, experimental studies (Davis et al. Science (1970) 169: 1329) in rats have shown that vitamin B6 deficiency can cause congenital malformations.

It has also been reported (Martindale) that oral contraceptives may cause vitamin B6 deficiency in some users. Thus it will be evident that users of oral contraceptives are at risk of developing a vitamin B6 deficiency. In particular, in females who become pregnant shortly after discontinuation of the use of an oral contraceptive, the risk of vitamin B6 deficiency is pronounced, especially because it usually takes a long time to restore vitamin B6 serum levels to normal.

Accordingly, in an especially preferred embodiment of the invention, the dosage units additionally contain at least 3 mg vitamin B6, more preferably from 5 to 250 mg vitamin B6. The term "vitamin B6" as used throughout this document encompasses any components which *in vivo* are converted into pyridoxal, pyridoxal phosphate or a pyridoxal salt. Particularly useful are vitamin B6 components that *in vivo* are converted for at least 10 mol% into pyridoxal, pyridoxal phosphate or a pyridoxal salt within 24 hours after administration. Inside living human and animal cells, pyridoxal phosphate and pyridoxamine phosphate are the biologically active forms of vitamin B6, acting as a co-enzymes in more than 100 biological reactions.

As regards deficiencies of folate, vitamin B12 and vitamin B6 it is noted that such deficiencies are commonly diagnosed on the basis of blood serum/plasma concentration. It is generally accepted that an adult human is deficient in folate if the blood serum concentration is less than 2.8 ng/ml. Similarly, vitamin B12 deficiency and vitamin B6 deficiency are diagnosed if the serum concentrations of vitamin B12 is below 211 pg/ml. and the plasma concentration of vitamin B6 (measured as pyridoxal-5-phosphate) is below 5 ng/ml. These reference values have been published by the Clinical Laboratories of the University of California, San Diego. The methods advocated by these clinical laboratories for the determination of folate, vitamin B12 and vitamin B6 levels are:
- Folate:: Chemiluminescent competitive method, CPT Code 82746
- Vitamin B12:: Chemiluminescent competitive method, CPT Code 82607
- Vitamin B6:: Radioimmunoassay, CPT Code 84207

The invention is further illustrated by means of the following examples.

### EXAMPLES

### Example 1

A contraceptive kit is prepared in the form of a strip comprising 28 pills, each pill weighing 0.25 grams. Of the 28 pills, 21 have composition A and 7 have composition B as indicated below:

| | Composition A | Composition B |
|---|---|---|
| Ethinyl estradiol | 30 µg | |
| Levonorgestrel | 150 µg | |
| Folinic acid | 0.5 mg | 0.5 mg |
| Vitamin B12 | 1 mg | 1 mg |
| Vitamin B6 | 50 mg | 50 mg |
| Excipient | remainder | remainder |

### Example 2

A pharmaceutical kit for use in a sequential contraceptive method is prepared in the form of a strip comprising 28 pills. The kit comprises 14 pills of composition A and 14 pills of composition B as described below:

| | Composition A | Composition B |
|---|---|---|
| Ethinyl estradiol | 30 µg | 30 µg |
| Levonorgestrel | | 150 µg |
| Folinic acid | 0.5 mg | 0.5 mg |
| Vitamin B 12 | 1 mg | 1 mg |
| Vitamin B6 | 50 mg | 50 mg |
| Excipient | remainder | remainder |

### Example 3

A group of 40 females is randomly divided across 2 groups of each 20 females. During a period of 4 months one group uses the contraceptive kit described in example 1. During the same period the other group uses the same kit with the sole exception that the pills in said kit do not contain folinic acid or vitamin B12 or vitamin B6.

Before the study is commenced as well as at the end of the study, serum concentrations of folate, vitamin B12 and vitamin B6 are determined using the aforementioned methods as published in 2002 by the Clinical Laboratories of the University of California, San Diego. Serum levels of these substances are found to not have significantly changed during the study in the group of females who received an oral contraceptive that did not contain added folinic acid, vitamin B12 or vitamin B6. A large fraction of the females in the other group, however, are found to exhibit significantly increased levels of folate, vitamin B 12 and/or vitamin B6.

At the beginning of the study some females are found to be deficient in folate, B12 and/or B6. Those females who exhibit such a deficiency and who received an oral contraceptive that had been reinforced with folinic acid, vitamin B12 and vitamin B6, are found to be no longer deficient in any of these 3 substances at the end of the study.

### Example 4

Example 3 is repeated but using the kit described in example 2 instead of the kit described in example 1.

Serum levels of folate, vitamin B12 and vitamin B6 are found to not have significantly changed during the study in the group of females who received an oral contraceptive that did not contain added folinic acid, vitamin B 12 or vitamin B6. A large fraction of the females in the other group are found to have significantly increased levels of folate, vitamin B12 and/or vitamin B6 at the end of the study.

The females who are found to be deficient in folate, vitamin B12 and/or vitamin B6 at the beginning of the study and who received an oral contraceptive that had been reinforced with these substances, are no longer deficient in any of these 3 substances at the end of the study.

## Claims

1. A kit for use in a hormonal contraceptive method or hormone replacement therapy in mammalian females, said kit comprising at least 10 oral dosage units containing:
a) at least 1 µg of one or more steroids selected from the group consisting of estrogens and progestogens;
b) at least 0.1 mg of one or more tetrahydrofolate components selected from the group consisting of (6S)-tetrahydrofolic acid, 5-methyl-(6S)-tetrahydrofolic acid, 5-formyl-(6S)-tetrahydrofolic acid, 10-formyl-(6R)-tetrahydrofolic acid, 5,10-methylene-(6R)-tetrahydrofolic acid, 5,10-methenyl-(6R)-tetrahydrofolic acid, 5-formimino-(6S)-tetrahydrofolic acid, pharmaceutically acceptable salts of these tetrahydrofolic acids and glutamyl derivatives of these tetrahydrofolic acids; and
c) at least 0.1 mg vitamin B12.

2. Kit according to claim 1, comprising at least 10 dosage units containing:
a) from 2 µg to 30 mg of the one or more steroids;
b) from 0.2 to 15 mg of the one or more tetrahydrofolate components and
c) from 0.2 to 20 mg vitamin B12.

3. Kit according to claim 1 or 2, wherein the kit additionally comprises one or more dosage units, preferably 3-8 dosage units that contain the one or more tetrahydrofolate components and vitamin B12 in the indicated amounts, but that do not contain an estrogen or a progestogen.

4. Kit according to any one of claims 1-3, wherein the one or more tetrahydrofolate components are selected from the group consisting of 5-formyl-tetrahydrofolic acid, 5-methyl-tetrahydrofolic acid, as well as pharmaceutically acceptable salts and glutamyl derivatives of these acids.

5. A kit comprising a plurality of steroid containing oral dosage units for use in a hormonal contraceptive method in mammalian females, said method comprising the at least once daily oral administration of one or more of the dosage units to a mammalian female so as to provide steroids in an effective amount to inhibit ovulation, and wherein the dosage units additionally contain at least 0.1 mg of one or more tetrahydrofolate components selected from the group consisting of (6S)-tetrahydrofolic acid, 5-methyl-(6S)-tetrahydrofolic acid, 5-formyl-(6S)-tetrahydrofolic acid, 10-formyl-(6R)-tetrahydrofolic acid, 5,10-methylene-(6R)-tetrahydrofolic acid, 5,10-methenyl-(6R)-tetrahydrofolic acid, 5-formimino-(6S)-tetrahydrofolic acid, pharmaceutically acceptable salts of these tetrahydrofolic acids and glutamyl derivatives of these tetrahydrofolic acids and at least 0.1 mg vitamin B12.

6. A kit comprising a plurality of steroid containing oral dosage units for use in hormone replacement therapy in peri-menopausal, menopausal or post menopausal mammalian females, said method comprising the at least once daily oral administration of one or more of the dosage units to the female so as to provide steroids in an effective amount to prevent or suppress symptoms of hypogonadism, and wherein the dosage units additionally contain at least 0.1 mg of one or more tetrahydrofolate components selected from the group consisting of (6S)-tetrahydrofolic acid, 5-methyl-(6S)-tetrahydrofolic acid, 5-formyl-(6S)-tetrahydrofolic acid, 10-formyl-(6R)-tetrahydrofolic acid, 5,10-methylene-(6R)-tetrahydrofolic acid, 5,10-methenyl-(6R)-tetrahydrofolic acid, 5-formimino-(6S)-tetrahydrofolic acid, pharmaceutically acceptable salts of these tetrahydrofolic acids and glutamyl derivatives of these tetrahydrofolic acids; and at least 0.1 mg vitamin B12.

7. Kit according to claim 5 or 6, wherein the steroids are selected from the group consisting of estrogens and progestogens.

8. Kit according to any one of claims 5-7, wherein the dosage units are administered to provide a therapeutically effective amount of the one or more tetrahydrofolate components to prevent or remedy deficiencies of a folate component.

9. Kit according to any one of claims 5-8, wherein the method comprises an administration regimen that provides to the female at least 18 consecutive daily dosages of:
a) from 2 µg to 30 mg of the one or more steroids;
b) from 0.2 to 15 mg of the one or more tetrahydrofolate components and
c) from 0.2 to 20 mg vitamin B12.

10. Kit according to any one of claims 5-9, wherein the one or more tetrahydrofolate components are selected from the group consisting of 5-formyl-tetrahydrofolic acid, 5-methyl-tetrahydrofolic acid, as well as pharmaceutically acceptable salts and glutamyl derivatives of these acids.

11. Kit according to any one of claims 5-10, wherein the method comprises an administration regimen that provides to the female at least 18 consecutive daily dosages of estrogen in an amount equivalent to 3-40 µg ethinyl estradiol and/or progestogen in an amount equivalent to 30 - 750 µg levonorgestrel.

12. Kit according to any one of claims 5-11, wherein the method comprises essentially continuous administration of the steroid containing dosage units during a time interval of at least 40 days, preferably at least 90 days.

13. Kit according to any one of claims 1-12, wherein the dosage units contain between 3 and 40 µg, preferably between 10 and 30 µg ethinyl estradiol.

14. Kit according to any one of claims 5-13, wherein the method comprises the essentially continuous administration of the one or more tetrahydrofolate components and vitamin B12 during a time interval of at least 40 days, preferably at least 90 days.

15. Kit according to claim 14, wherein the method comprises an interval of 3-8 days during which the one or more tetrahydrofolate components and vitamin B12 are administered, but during which no estrogen or progestogen is administered.

16. Kit according to any one of claims 1-15, wherein the one or more tetrahydrofolate components are selected from the group consisting of folinic acid, pharmaceutically acceptable salts of folinic acid, glutamyl derivatives of folinic acid and precursors of these substances.

17. Kit according to any one of claims 1-16, wherein the dosage units contain at least 3 mg vitamin B6, more preferably from 5 to 250 mg vitamin B6.

## Patentansprüche

1. Set zur Verwendung in einem hormonalen Empfängnisverhütungsverfahren oder einer Hormonersatztherapie bei weiblichen Säugern, wobei das Set mindestens 10 orale Dosiseinheiten aufweist, die enthalten:
a) mindestens 1 µg eines oder mehrerer Steroide, die aus der Gruppe ausgewählt sind, die aus Östrogenen und Progestogenen besteht;
b) mindestens 0,1 mg einer oder mehrerer Tetrahydrofolatkomponenten, die aus der Gruppe ausgewählt sind, die besteht aus (6S)-Tetrahydrofolsäure, 5-Methyl-(6S)-tetrahydrofolsäure, 5-Formyl-(6S)-tetrahydrofolsäure, 10-Formyl-(6R)-tetrahydrofolsäure, 5,10-Methylen-(6R)-tetrahydrofolsäure, 5,10-Methenyl-(6R)-tetrahydrofolsäure, 5-Formimino-(6S)-tetrahydrofolsäure, pharmazeutisch akzeptablen Salzen dieser Tetrahydrofolsäuren und Glutamylderivaten dieser Tetrahydrofolsäuren; und
c) mindestens 0,1 mg Vitamin B12.

2. Set nach Anspruch 1, das mindestens 10 Dosiseinheiten aufweist, die enthalten:
a) 2 µg bis 30 mg des einen oder der mehreren Steroide;
b) 0,2 bis 15 mg der einen oder der mehreren Tetrahydrofolatkomponenten und
c) 0,2 bis 20 mg Vitamin B12.

3. Set nach Anspruch 1 oder 2, wobei das Set zusätzlich eine oder mehrere Dosiseinheiten, bevorzugt 3 bis 8 Dosiseinheiten aufweist, welche die eine oder die mehreren Tetrahydrofolatkomponenten und Vitamin B12 in den angegebenen Mengen enthalten, jedoch kein Östrogen oder Progestogen enthalten.

4. Set nach einem der Ansprüche 1 bis 3, wobei die eine oder die mehreren Tetrahydrofolatkomponenten aus der Gruppe ausgewählt sind, die besteht aus 5-Formyl-tetrahydrofolsäure, 5-Methyl-tetrahydrofolsäure sowie pharmazeutisch akzeptablen Salzen und Glutamylderivaten dieser Säuren.

5. Set, das eine Vielzahl von Steroid enthaltenden oralen Dosiseinheiten aufweist, zur Verwendung in einem hormonalen Empfängnisverhütungsverfahren bei weiblichen Säugern, wobei das Verfahren die mindestens einmal täglich erfolgende orale Verabreichung einer oder mehrerer der Dosiseinheiten an einen weiblichen Säuger aufweist, um Steroide in einer wirksamen Menge zur Ovulationshemmung bereitzustellen, und wobei die Dosiseinheiten zusätzlich mindestens 0,1 mg einer oder mehrerer Tetrahydrofolatkomponenten enthalten, die aus der Gruppe ausgewählt sind, die besteht aus (6S)-Tetrahydrofolsäure, 5-Methyl-(6S)-tetrahydrofolsäure, 5-Formyl-(6S)-tetrahydrofolsäure, 10-Formyl-(6R)-tetrahydrofolsäure, 5,10-Methylen-(6R)-tetrahydrofolsäure, 5,10-Methenyl-(6R)-tetrahydrofolsäure, 5-Formimino-(6S)-tetrahydrofolsäure, pharmazeutisch akzeptablen Salzen dieser Tetrahydrofolsäuren und Glutamylderivaten dieser Tetrahydrofolsäuren und mindestens 0,1 mg Vitamin B12.

6. Set, das eine Vielzahl von Steroid enthaltenden oralen Dosiseinheiten aufweist, zur Verwendung in der Hormonersatztherapie bei perimenopausalen, menopausalen oder postmenopausalen weiblichen Säugern, wobei das Verfahren die mindestens einmal täglich erfolgende orale Verabreichung einer oder mehrerer der Dosiseinheiten an einen weiblichen Säuger aufweist, um Steroide in einer wirksamen Menge zur Verhinderung oder Unterdrückung von Symptomen von Hypogonadismus bereitzustellen, und wobei die Dosiseinheiten zusätzlich mindestens 0,1 mg einer oder mehrerer Tetrahydrofolatkomponenten enthalten, die aus der Gruppe ausgewählt sind, die besteht aus (6S)-Tetrahydrofolsäure, 5-Methyl-(6S)-tetrahydrofolsäure, 5-Formyl-(6S)-tetrahydrofolsäure, 10-Formyl-(6R)-tetrahydrofolsäure, 5,10-Methylen-(6R)-tetrahydrofolsäure, 5,10-Methenyl-(6R)-tetrahydrofolsäure, 5-Formimino-(6S)-tetrahydrofolsäure, pharmazeutisch akzeptablen Salzen dieser Tetrahydrofolsäuren und Glutamylderivaten dieser Tetrahydrofolsäuren; und mindestens 0,1 mg Vitamin B12.

7. Set nach Anspruch 5 oder 6, wobei die Steroide aus der Gruppe ausgewählt sind, die aus Östrogenen und Progestogenen besteht.

8. Set nach einem der Ansprüche 5 bis 7, wobei die Dosiseinheiten verabreicht werden, um eine therapeutisch wirksame Menge der einen oder der mehreren Tetrahydrofolatkomponenten bereitzustellen, um einen Mangel an einer Folatkomponente zu verhindern oder zu beheben.

9. Set nach einem der Ansprüche 5 bis 8, wobei das Verfahren ein Verabreichungsregime aufweist, das den weiblichen Säuger mit mindestens 18 aufeinanderfolgenden Tagesdosen versorgt von:
a) 2 µg bis 30 mg des einen oder der mehreren Steroide;
b) 0,2 bis 15 mg der einen oder der mehreren Tetrahydrofolatkomponenten und
c) 0,2 bis 20 mg Vitamin B12.

10. Set nach einem der Ansprüche 5 bis 9, wobei die eine oder die mehreren Tetrahydrofolatkomponenten aus der Gruppe ausgewählt sind, die besteht aus 5-Formyltetrahydrofolsäure, 5-Methyltetrahydrofolsäure sowie pharmazeutisch akzeptablen Salzen und Glutamylderivaten dieser Säuren.

11. Set nach einem der Ansprüche 5 bis 10, wobei das Verfahren ein Verabreichungsregime umfasst, das den weiblichen Säuger versorgt mit mindestens 18 aufeinanderfolgenden Tagesdosen Östrogen in einer Menge, die 3 bis 40 µg Ethinylöstradiol äquivalent ist, und/oder Progestogen in einer Menge, die 30 bis 750 µg Levonorgestrel äquivalent ist.

12. Set nach einem der Ansprüche 5 bis 11, wobei das Verfahren die im Wesentlichen kontinuierliche Verabreichung der Steroid enthaltenden Dosiseinheiten während eines Zeitraums von mindestens 40 Tagen, bevorzugt mindestens 90 Tagen umfasst.

13. Set nach einem der Ansprüche 1 bis 12, wobei die Dosiseinheiten zwischen 3 und 40 µg, bevorzugt zwischen 10 und 30 µg Ethinylöstradiol enthalten.

14. Set nach einem der Ansprüche 5 bis 13, wobei das Verfahren die im Wesentlichen kontinuierliche Verabreichung der einen oder der mehreren Tetrahydrofolatkomponenten und von Vitamin B12 während eines Zeitraums von mindestens 40 Tagen, bevorzugt mindestens 90 Tagen umfasst.

15. Set nach Anspruch 14, wobei das Verfahren einen Zeitraum von 3 bis 8 Tagen umfasst, in dem die eine oder die mehreren Tetrahydrofolatkomponenten und Vitamin B12 verabreicht werden, in dem jedoch kein Östrogen oder Progestogen verabreicht wird.

16. Set nach einem der Ansprüche 1 bis 15, wobei die eine oder die mehreren Tetrahydrofolatkomponenten aus der Gruppe ausgewählt sind, die besteht aus Folinsäure, pharmazeutisch akzeptablen Salzen von Folinsäure, Glutamylderivaten von Folinsäure und Vorläufern dieser Substanzen.

17. Set nach einem der Ansprüche 1 bis 16, wobei die Dosiseinheiten mindestens 3 mg Vitamin B6, stärker bevorzugt 5 bis 250 mg Vitamin B6 enthalten.

## Revendications

1. Kit destiné à être utilisé dans un procédé de contraception hormonale ou une hormonothérapie substitutive chez des femelles de mammifères, ledit kit comprenant au moins 10 unités de prise orales contenant :
a) au moins 1 µg d'un ou plusieurs stéroïdes choisis dans le groupe consistant en les estrogènes et les progestogènes ;
b) au moins 0,1 mg d'un ou plusieurs composants tétrahydrofolates choisis dans le groupe consistant en l'acide (6S)-tétrahydrofolique, l'acide 5-méthyl-(6S)-tétrahydrofolique, l'acide 5-formyl-(6S)-tétrahydrofolique, l'acide 10-formyl-(6R)-tétrahydrofolique, l'acide 5,10-méthylène-(6R)-tétrahydrofolique, l'acide 5,10-méthényl-(6R)-tétrahydrofolique, l'acide 5-formimino-(6S)-tétrahydrofolique, les sels pharmaceutiquement acceptables de ces acides tétrahydrofoliques et les dérivés glutamyle de ces acides tétrahydrofoliques ; et
c) au moins 0,1 mg de vitamine B12.

2. Kit selon la revendication 1 comprenant au moins 10 unités de prise contenant :
a) de 2 µg à 30 mg d'un ou plusieurs stéroïdes ;
b) de 0,2 à 15 mg d'un ou plusieurs composants tétrahydrofolates et
c) de 0,2 à 20 mg de vitamine B12.

3. Kit selon la revendication 1 ou 2 où le kit comprend en outre une ou plusieurs unités de prise, de préférence 3-8 unités de prise qui contiennent le ou les composants tétrahydrofolates et la vitamine B12 en les quantités indiquées, mais qui ne contiennent pas d'estrogène ou de progestogène.

4. Kit selon l'une quelconque des revendications 1-3 où le ou les composants tétrahydrofolates sont choisis dans le groupe consistant en l'acide 5-formyl-tétrahydrofolique, l'acide 5-méthyl-tétrahydrofolique, ainsi que les sels pharmaceutiquement acceptables et les dérivés glutamyle de ces acides.

5. Kit comprenant une pluralité d'unités de prise orales contenant des stéroïdes destiné à être utilisé dans un procédé de contraception hormonale chez des femelles de mammifères, ledit procédé comprenant l'administration orale au moins une fois par jour d'une ou plusieurs des unités de prise à une femelle de mammifère de manière à fournir des stéroïdes en une quantité efficace pour inhiber l'ovulation, et où les unités de prise contiennent en outre au moins 0,1 mg d'un ou plusieurs composants tétrahydrofolates choisis dans le groupe consistant en l'acide (6S)-tétrahydrofolique, l'acide 5-méthyl-(6S)-tétrahydrofolique, l'acide 5-formyl-(6S)-tétrahydrofolique, l'acide 10-formyl-(6R)-tétrahydrofolique, l'acide 5,10-méthylène-(6R)-tétrahydrofolique, l'acide 5,10-méthényl-(6R)-tétrahydrofolique, l'acide 5-formimino-(6S)-tétrahydrofolique, les sels pharmaceutiquement acceptables de ces acides tétrahydrofoliques et les dérivés glutamyle de ces acides tétrahydrofoliques et au moins 0,1 mg de vitamine B12.

6. Kit comprenant une pluralité d'unités de prise orales contenant des stéroïdes destiné à être utilisé dans une hormonothérapie substitutive chez des femelles de mammifères péri-ménopausées, ménopausées ou post-ménopausées, ledit procédé comprenant l'administration orale au moins une fois par jour d'une ou plusieurs des unités de prise à la femelle de manière à fournir des stéroïdes en une quantité efficace pour prévenir ou supprimer les symptômes d'hypogonadisme, et où les unités de prise contiennent en outre au moins 0,1 mg d'un ou plusieurs composants tétrahydrofolates choisis dans le groupe consistant en l'acide (6S)-tétrahydrofolique, l'acide 5-méthyl-(6S)-tétrahydrofolique, l'acide 5-formyl-(6S)-tétrahydrofolique, l'acide 10-formyl-(6R)-tétrahydrofolique, l'acide 5,10-méthylène-(6R)-tétrahydrofolique, l'acide 5,10-méthényl-(6R)-tétrahydrofolique, l'acide 5-formimino-(6S)-tétrahydrofolique, les sels pharmaceutiquement acceptables de ces acides tétrahydrofoliques et les dérivés glutamyle de ces acides tétrahydrofoliques ; et au moins 0,1 mg de vitamine B12.

7. Kit selon la revendication 5 ou 6 où les stéroïdes sont choisis dans le groupe consistant en les estrogènes et les progestogènes.

8. Kit selon l'une quelconque des revendications 5-7 où les unités de prise sont administrées pour fournir une quantité thérapeutiquement efficace du ou des composants tétrahydrofolates pour prévenir ou guérir des carences en un composant folate.

9. Kit selon l'une quelconque des revendications 5-8 où le procédé comprend un schéma d'administration qui fournit à la femelle au moins 18 dosages quotidiens consécutifs de :
a) de 2 µg à 30 mg d'un ou plusieurs stéroïdes ;
b) de 0,2 à 15 mg d'un ou plusieurs composants tétrahydrofolates et
c) de 0,2 à 20 mg de vitamine B12.

10. Kit selon l'une quelconque des revendications 5-9 où le ou les composants tétrahydrofolates sont choisis dans le groupe consistant en l'acide 5-formyl-tétrahydrofolique, l'acide 5-méthyl-tétrahydrofolique, ainsi que les sels pharmaceutiquement acceptables et les dérivés glutamyle de ces acides.

11. Kit selon l'une quelconque des revendications 5-10 où le procédé comprend un schéma d'administration qui fournit à la femelle au moins 18 dosages quotidiens consécutifs d'estrogène en une quantité équivalant à 3-40 µg d'éthynyl estradiol et/ou de progestogène en une quantité équivalant à 30-750 µg de levonorgestrel.

12. Kit selon l'une quelconque des revendications 5-11 où le procédé comprend l'administration sensiblement continue des unités de prise contenant des stéroïdes pendant un intervalle de temps d'au moins 40 jours, de préférence d'au moins 90 jours.

13. Kit selon l'une quelconque des revendications 1-12 où les unités de prise contiennent entre 3 et 40 µg, de préférence entre 10 et 30 µg d'éthynyl estradiol.

14. Kit selon l'une quelconque des revendications 5-13 où le procédé comprend l'administration sensiblement continue d'un ou plusieurs composants tétrahydrofolates et de vitamine B12 pendant un intervalle de temps d'au moins 40 jours, de préférence d'au moins 90 jours.

15. Kit selon la revendication 14 où le procédé comprend un intervalle de 3-8 jours pendant lequel le ou les composants tétrahydrofolates et la vitamine B12 sont administrés, mais pendant lequel aucun estrogène ou progestogène n'est administré.

16. Kit selon l'une quelconque des revendications 1-15 où le ou les composants tétrahydrofolates sont choisis dans le groupe consistant en l'acide folinique, les sels pharmaceutiquement acceptables de l'acide folinique, les dérivés glutamyle de l'acide folinique et les précurseurs de ces substances.

17. Kit selon l'une quelconque des revendications 1-16 où les unités de prise contiennent au moins 3 mg de vitamine B6, de préférence encore de 5 à 250 mg de vitamine B6.
